(19) Europäisches Patentamt
European Patent Office
Office européen des brevets

(11) Veröffentlichungsnummer: **0 273 253 A2**

# (12) EUROPÄISCHE PATENTANMELDUNG

(21) Anmeldenummer: 87118209.3

(22) Anmeldetag: 09.12.87

(51) Int. Cl.⁴: **A61K 31/55**

(30) Priorität: 13.12.86 DE 3642648

(43) Veröffentlichungstag der Anmeldung: 06.07.88 Patentblatt 88/27

(84) Benannte Vertragsstaaten: AT BE CH DE FR GB IT LI LU NL SE

(71) Anmelder: BOEHRINGER INGELHEIM KG
D-6507 Ingelheim am Rhein(DE)

(84) BE CH DE FR IT LI LU NL SE AT

(71) Anmelder: Boehringer Ingelheim International G.m.b.H
D-6507 Ingelheim am Rhein(DE)

(84) GB

(72) Erfinder: Brantl, Victor, Dr.
Frauenplatz 10
D-8000 München 2(DE)

## (54) Verwendung von 6-Allyl-2-amino-5,6,7,8-tetrahydro-4H-thiazolo-[5,4-d]azepin zur Freisetzung von Wachstumshormon.

(57) Die Erfindung betrifft die Verwendung von 6-Allyl-2-amino-5,6,7,8-tetrahydro-4H-thiazolo[4,5-d]azepin sowie dessen pharmakologische Säureadditionssalz zur Herstellung von Arzneimitteln zur Freisetzung von Wachstumshormon.

EP 0 273 253 A2

Xerox Copy Centre

# Verwendung von 6-Allyl-2-amino-5,6,7,8-tetrahydro-4H-thiazolo-[5,4-d]azepin zur Freisetzung von Wachstumshormon

Die Erfindung betrifft die Verwendung eines Thiazoloazepin-Derivats sowie seiner pharmakologisch verträglichen Säureadditionssalze zur Freisetzung von Wachstumshormon, z.B. zur Behandlung von Wachstumsstörungen.

In der deutschen Offenlegungsschrift Nr. 20 40 510 sind Thiazolo-und Oxazolo-Derivate der allgemeinen Formel

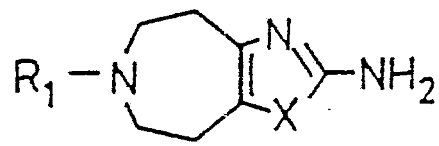

in der $R_1$ ein Wasserstoffatom, eine gegebenenfalls durch ein Hydroxylgruppe substituierte Alkylgruppe mit 1 bis 4 Kohlenstoffatomen, eine gegebenenfalls durch ein Halogenatom, eine Methyl-oder Methoxygruppe substituierte Benzoylgruppe oder eine Allylgruppe und X ein Sauerstoff oder Schwefelatom bedeuten sowie deren physiologisch verträgliche Säureadditionssalze mit anorganischen oder organischen Säuren beschrieben.

Aus der Offenlegungsschrift ist weiterhin bekannt, daß die Verbindungen der allgemeinen Formel I wertvolle pharmakologische Eigenschaften aufweisen, wobei in Abhängigkeit von X sowohl hustenstillende wie auch blutdrucksenkende Eigenschaften beschrieben sind. Eine Retardform zur oralen Behandlung der Hypertonie und von Angina Pectoris ist in der DE-OS 28 36 387 offenbart. Die US-PS 4 400 378 beschreibt ferner auch eine Antiglaucoma-Wiksamkeit von Verbindungen der allgemeinen Formel I.

Weiterhin ist das 6-Allyl-2-amino-5,6,7,8-tetrahydro-4H-thiazolo[4,5-d]azepin als B-HT 920 in zahlreichen wissenschaftlichen Publikationen beschrieben. Anmeldungen zu einer zweiten medizinischen Indikation erfolgten in der DE-OS 35 02 365 als Mittel zur Senkung des Prolactin-Serum-Spiegels und in der DE-OS 35 03 963 als Mittel zur Behandlung des Parkinsonismus. Aus verschiedenen Publikationen und Patentanmeldungen sind bereits Verbindungen bekannt, die eine Freisetzung des Wachstumsfaktors bewirken (Lit.: F.X. Coude et al., Irends in Biotechnology 1984, Volume 2, S. 83 ff; M.O. Thorner in "The Lancet", July 16, 1983, S. 119 ff; EP-OS 0 136 475). Hierbei handelt es sich um Polypeptide, in der Regel mit einer Sequenz aus 40 Aminosäuren. In der angelsächsischen Literatur werden diese Peptide auch als "Growth Hormone Releasing Factor" (GRF) bezeichnet. Die Herstellung dieser Verbundungen kann durch konventionelle chemische Synthese oder ab auch gentechnologische Verfahren erfolgen. Infolge der komplexen Struktur dieser Peptide ist es bislang nicht möglich, diese mit Hilfe einer konventionellen Synthese in größeren Mengen herzustellen, auch gentechnologische Verfahren ermöglichen noch kein kostengünstigeres Herstellungsverfahren in größerem Maßstab.

Überraschenderweise wurde gefunden, daß B-HT 920 beim Meschen eine Freisetzung des Wachstumshormons bewirkt. B-HT 920 sowie seine verträglichen Säureadditionssalze eignen sich zur Herstellung eines Arzneimittels zur Behandlung von Wachstumsstörungen.Es kann zur Behandlung von Krankheiten eingesetzt werden, die auf einer verminderten Ausschüttung des Wachstumshormons beruhen, wie z.B. Wachstummstörungen bei Kindern, so z.B. bei Minderwuchs, ebenso bei vermindertem Stoffwechsel, wie z.B. Maluntrition, Kachexie bei Tumoren oder der Chemotherapie; chronische Anoxie durch respiratorische Insuffizienz oder Cardiopathie, Niereninsuffizienz. Weitere Indikationsgebiete sind Knochenfrakturen, Verbrennungen, Wundheilung und Beschleunigung der Blutbildung.

Zu diagnostischen Zwecken kann B-HT 920 eingesetzt werden, um die Freisetzung von Wachstumshormon zu stimulieren und um so festzustellen, ob ausreichend Wachstumshormon in der Hypophose vorhanden ist.

Zur Freisetzung des Wachstumshormon läßt sich B-HT 920 und dessen verträgliche Säureadditionssalze (z.B. B-HT 920 $Cl_2$) in die üblichen galenischen Zubereitungsformen für orale, rektale, parenterale oder transdermale Anwendungen einarbeiten.

Die orale Einzeldosis beim Menschen liegt normalerweise zwischen 0.05 mg und 0,3 mg, bevorzugt 0.1 bis 0.25 mg. Bei einer Mehrfachapplikation über den Tag verteilt kann die Gesamtdosis zwischen 0.6 und 1.0 mg liegen.

Im folgenden werden klinische Untersuchungen über die Freisetzung von Wachstumshormon von B-HT

920 $Cl_2$ beschrieben.

Die Versuche werden mit sech männlichen Probanden im Alter von 21 bis 46 Jahren durchgeführt. Jeder Proband erhielt 0.15 bis 0.2 mg B-HT 920 $Cl_2$ als Tablette.

Die Bestimmungen des Wachstumshormons im Serum erfolgte mit Hilfe eines radioimunologischen Standardverfahrens.

Die Serumspiegel wurden vor und nach der Applikation bestimmt. Die Blutentnahmen erfolgten über eine Venendauerkanüle, die 1 Stunde vor Substanzgabe und Entnahme des O-Wertes gelegt wurden. Die Meßergebnisse sind in Tabelle I aufgeführt. Die Meßwerte sind in ng/ml angegeben.

| | | | Stunden nach Medikation | | | | |
|---|---|---|---|---|---|---|---|
| Dosis B-HT920$Cl_2$ | Proband | 0-Wert t=0 | 1 | 2 | 3 | 5 | 8 |
| 0,15 mg | 1 | 0,3 | 0,5 | 0,3 | 3,5 | 4,4 | 0,4 |
| 0,15 mg | 2 | 0,4 | 0,3 | 0,3 | 0,5 | 3,3 | 0,3 |
| 0,20 mg | 3 | 1,5 | 0,4 | 2,5 | 9,5 | 1,0 | 0,5 |
| 0,15 mg | 4 | 0,3 | 0,32 | 1,7 | 0,3 | 0,2 | 0,2 |
| 0,15 mg | 5 | 0,3 | 7,3 | 0,6 | 0,4 | 0,2 | 0,3 |
| 0,15 mg | 6 | 0,4 | 20,0 | 12,4 | 1,4 | 0,4 | 0,4 |

Die Substanz ist sehr gut verträglich, unerwünschte Wirkung, wie z.B. Mundtrockenheit, Müdigkeit oder Schwindelgefühle treten nicht auf.

Die nachfolgenden Beispiele beschreiben die Herstellung einiger pharmazeutischer Zubereitungsformen:

Beispiel I

Dragéekern

Zusammensetzung: 1 Dragéekern enthält

| | |
|---|---|
| B-HT 920 $Cl_2$ | 100 µg |
| Milchzucker | 38,45 mg |
| Maisstärke | 10,0 mg |
| Gelatine | 1,0 mg |
| Magnesiumstearat | 0,5 mg |

Herstellungsverfahren

Die Mischung der Wirksubstanz mit Milchzucker und Maisstärke wird mit einer 10-%igen wäßrigen Gelatinelösung durch Sieb 1 mm granuliert, bei 40°C getrocknet und nochmals durch obiges Sieb gerieben. Das so erhaltene Granulat wird mit Magnesiumstearat gemischt und zu Dragéekernen verpreßt. Die Herstellung soll in abgedunkelten Räumen vorgenommen werden.

Kerngewicht: 50 mg
Stempel: 5 mm, gewölbt

Die so erhaltenen Dragéekerne werden nach bekanntem Verfahren mit einer Hülle überzogen, die im wesentlichen aus Zucker un Talkum besteht. Die fertigen Dragées werden mit Hilfe von Bienenwachs poliert.
Dragéegewicht : 100 mg

Beispiel II

Suppositorien 1 Zäpfchen enthält:
B-HT 920 $Cl_2$ 100,0 µg
Zäpfchenmass (z.B. Witepsol W 45) 1690,0 mg

Herstellungsverfahren:

Die feingepulverte Substanz wird mit Hilfe eines Eintauchhomogenisators in die geschmolzene und auf 40°C abgekühlte Zäpfchenmass eingerührt. Die Masse wird bei 35°C in leicht vorgekühlte Formen ausgegossen.

Beispiel III

Ampullen mit 200 µg B-HT 920 1 Ampule enthält:

| | |
|---|---|
| B-HT 920 | 200 µg |
| Zitronensäure | 7,0 mg |
| Natriumphosphat sek. $2H_2O$ | 3,0 mg |
| Natriumpyrosulfit | 1,0 mg |
| Dest. Wasser | ad 1,0 ml |

Herstellungsverfahren:

In ausgekochtem und unter $CO_2$-Begasung abgekühltem Wasser werden nacheinander die Puffersubstanzen, die Wirksubstanz sowie Natriumpyrosulfit gelöst. Man füllt mit abgekochtem Wasser auf das gegebene Volumen auf und filtriert pyrogenfrei.

Abfüllung: in braune Ampullen unter Schutzbegasung Sterilisation: 20 Minuten bei 120°C

Die Herstellung und Abfüllung der Ampullenlösung muß in abgedunkelten Räumen vorgenommen werden.

Beispiel IV

Dragées mit 1 mg B-HT 920 $Cl_2$1 Dragéekern enthält:

| | |
|---|---|
| B-HT 920 $Cl_2$ | 100 µg |
| Milchzurker | 36,0 mg |
| Maisstärke | 12,4 mg |
| Gelatine | 1,0 mg |
| Magnesiumstearat | 0,5 mg |

Herstellungsverfahren:Analogo Beispiel I.

| | |
|---|---|
| Kerngewicht: | 50 mg |
| Stempel: | 5 mm, gewölbt |
| Dragéegewicht: | 100 mg |

Beispiel V

Gelatine-Steckkapseln mit 250 µg B-HT 920 $Cl_2$1 Kapsel enthält:

| | |
|---|---|
| B-HT 920 $Cl_2$ | 250 µg |
| Maisstärke | 85,7 mg |

Herstellungsverfahren:

Die Substanzen werden intensiv gemischt und in Opak-Kapseln geeigneter Größe abgefüllt.

Herstellungsbeispiel für eine transdermale Applikationsform :

Zusammensetzung:

| | |
|---|---|
| 9.744 g | Eudragit $^R$ E 30 D (Fa. Röhm, Darmstadt) |
| 0.600 g | Eudragit $^R$ E 100 (Fa. Röhm, Darmstadt) |
| 1.656 g | (B-HT 920) |
| 12.000 g | Feststoff |
| 6.000 g | Aceton |
| 22.000 g | Methanol |
| 100.000 g | Lösung |

Das Eudragit $^R$ E 100 wird in Aceton vorgelöst, anschließend wird das Eudragit $^R$ E 30 D und die Hälfte des Methanols zugegeben und gerührt. Nachdem eine homogene Lösung entstanden ist, wird der Wirkstoff und das restliche Methanol portionsweise eingetragen. Die so erhaltene Lösung wird auf ein Filmziehgerät (d. Fa. Erichsen) auf eine vorbereitete wirkstoffundurchlässige Rückschicht gegossen und bei einer

Rakelstellung von 0,6 mm abgezogen. Nach 10 minütigem Trocknen wird eine weitere und dann noch eine dritte Schicht bei gleicher Rakelstellung aufgetragen. Nach dem Trocknen erhält man einen Film mit einer Schichtdicke von 100 µm. Anschließend wird der Film konfektioniert und zum Gebrauch mit einem geeigneten Haftpflaster auf der Haut befestigt.

**Ansprüche**

1. Verwendung von 6-Allyl-2-amino-5,6,7,8-tetrahydro-4H-thiazolo[5,4-d]-azepin und dessen pharmakologisch verträgliche Säureadditionssalze zur Herstellung von Arzneimitteln zur Freisetzung von Wachstumshormon.

2. Verwendung von 6-Allyl-2-amino-5,6,7,8-tetrahydro-4H-thiazolo[5,4-d]-azepin und dessen pharmakologisch verträgliche Säureadditionssalze zur Herstellung von Arzneimitteln zur Behandlung von Krankheiten, hervorgerufen durch eine verminderte Ausschüttung von Wachstumshormon.